# EUROPÄISCHE PATENTSCHRIFT

(11) **EP 1 123 330 B1**
(45) Veröffentlichungstag und Bekanntmachung des Hinweises auf die Patenterteilung: **03.03.2004**
(21) Anmeldenummer: 99948879.4
(22) Anmeldetag: 28.09.1999
(51) Int. Cl.: C08F 251/00, C08F 261/04, C08F 283/06, C08F 283/02, C08F 220/06, A61L 15/00, A61F 13/15

(54) **VERFAHREN ZUR HERSTELLUNG VON WASSERQUELLBAREN HYDROPHILEN POLYMEREN, DIE POLYMERE SOWIE DEREN VERWENDUNG**
METHOD FOR PRODUCING WATER-SWELLABLE HYDROPHILIC POLYMERS, SAID POLYMERS AND USE THEREOF
PROCEDE PERMETTANT DE PRODUIRE DES POLYMERES HYDROPHILES GONFLANT A L'EAU, POLYMERES AINSI OBTENUS ET LEUR UTILISATION

(30) Priorität: 08.10.1998 DE 19846413
(43) Veröffentlichungstag der Anmeldung: 16.08.2001
(73) Patentinhaber: BASF AKTIENGESELLSCHAFT, 67056 Ludwigshafen (DE)
(72) Erfinder: DENTLER, Joachim, D-63486 Bruchköbel (DE); HERFERT, Norbert, D-63674 Altenstadt (DE); SCHLIWA, Rudolf, D-63755 Alzenau (DE); STÜVEN, Uwe, D-65812 Bad Soden (DE); ENGELHARDT, Fritz, Chesapeake, VA 23320 (US)
(86) Internationale Anmeldenummer: PCT/EP1999/007176
(87) Internationale Veröffentlichungsnummer: WO 2000/022017

(56) Entgegenhaltungen:
- WO-A-97/06190
- US-A- 4 985 514
- US-A- 5 250 640

## Beschreibung

Die vorliegende Erfindung betrifft ein Verfahren zur Herstellung von wasserquellbaren hydrophilen Polymerisaten, die hiernach erhaltenen Polymere sowie die Verwendung dieser Polymerisate.

Hydrophile Hydrogele, die durch Polymerisation ungesättigter Säuren, wie beispielsweise Acrylsäure, Methacrylsäure, Acrylamidopropansulfonsäure usw., in Gegenwart geringer Mengen mehrfach olefinisch ungesättigter Verbindungen erhalten werden können, sind bereits als superabsorbierende Polymere bekannt.

Weiterhin sind auch hydrophile Hydrogele bekannt, die durch Pfropfcopolymerisation olefinisch ungesättigter Säuren auf unterschiedliche Matrices, wie beispielsweise Polysaccharide, Polyalkylenoxide sowie deren Derivate, zugänglich sind.

Die genannten Hydrogele zeichnen sich durch ein hohes Aufnahmevermögen für Wasser und wäßrige Lösungen aus und finden daher bevorzugt Anwendung als Absorptionsmittel in Hygieneartikeln.

Die Herstellung solch wasserquellbarer hydrophiler Polymerer erfolgt in der Regel durch radikalische Polymerisation in wäßriger Lösung, die die Monomeren, sowie gegebenenfalls Pfropfgrundlage und Vernetzer enthält.

Für die Verwendung der wasserquellbaren hydrophilen Polymere im Hygiene- und Sanitärbereich werden Polymerisate erzeugt, deren Neutralisationsgrad zwischen 60 und 85 Mol-% bezogen auf die polymerisierten, Säuregruppen enthaltenden Monomereinheiten, beträgt, so daß bei der Verwendung hautneutral wirkende Hydrogele gebildet werden.

Die Einstellung des Neutralisationsgrades wird in der Regel vor der Polymerisation vorgenommen, da so die technisch schwierig durchzuführende Neutralisation eines sauren, hochviskosen Hydrogels umgangen wird. Nun zeigt aber die Polymerisation von z.B. Acrylsäure im neutralen pH-Bereich eine niedrigere Polymerisationsgeschwindigkeit und führt zu niedrigeren Molekulargewichten als die Polymerisation im sauren Bereich. Dies wird durch die elektrostatische Abstoßung zwischen der zuletzt eingebauten Monomereinheit und der neu einzubauenden Monomereinheit erklärt, die im Falle der Polymerisation im sauren pH-Bereich nicht oder nur gering auftritt, da die Monomereinheiten in der ungeladenen, sauren Form vorliegen.

Mit dem Trend zu immer dünner werdenden Windelkonstruktionen werden an die wasserquellbaren hydrophilen Polymeren zunehmend steigende Anforderungen an Absorptionsvermögen, Gelstärke, Gelpermeabilität und Restgehalt an Extrahierbaren gestellt.

Die gewünschte Kombination von hoher Absorption, hoher Gelstärke, hohe Gelpermeabilität und niedrige Restgehalte an Extrahierbaren kann nur durch eine Polymerisation erreicht werden, bei der möglichst hohe Molekulargewichte der primären Polymerketten erzielt werden. Das bevorzugte Herstellungsverfahren für solche Produkte ist daher die Polymerisation in wäßriger Lösung, bei der die säuregruppentragenden Monomereinheiten in der Monomerlösung nur zum Teil oder gar nicht vorneutralisiert vorliegen. Der Neutralisationsgrad der Säuregruppen tragenden Monomere beträgt hierbei bevorzugt 0 bis 40 Mol.-% und besonders bevorzugt 0 - 25 mol.-%.

Durch die Polymerisation und anschließende Grobverkleinerung erhält man saure Hydrogel-Teilchen, die auf den gewünschten Endneutralisationsgrad von 60 - 85 Mol-% bezogen auf Säuregruppen tragende Monomereinheiten durch Neutralisation dieser Säuregruppen tragenden Monomereinheiten gebracht werden müssen. Diese Neutralisation ist ein technisch schwierig durchzuführender Prozeß, an den besondere Anforderungen gestellt werden. Zum einen darf das Gel bei dem Inkontaktbringen mit dem Neutralisationsmittel nicht zu sehr geschert werden, um einen Anstieg der Extrahierbaren zu vermeiden, der sich negativ auf die Produkteigenschaften des Endproduktes auswirkt und somit unerwünscht ist. Zum anderen muß die Neutralisation vollständig homogen erfolgen, um ein ausreichend hohes Trocknungsverfahren der Gelpartikel zu erzielen. Saure Hydrogelteilchen mit einem geringen Neutralisationsgrad sind nämlich ausgesprochen klebrig und sind nicht imstande, bei der nachfolgenden Bandtrocknung ein lockeres Haufwerk zu bilden, welches für die Erzielung hoher Trocknungsleistungen erforderlich ist.

Die nachträgliche Neutralisation von sauren Hydrogelen ist prinzipiell bekannt.

DE-A-26 12 846 offenbart ein Verfahren zur Herstellung eines wasserabsorbierenden Harzes, wobei wenigstens eine Stärke und/oder Cellulose mit wenigstens einem wasserlöslichen Monomeren mit einer polymerisierbaren Doppelbindung und einem Vernetzer polymerisiert wird. Die erhaltenen Polymeren werden mit Basen neutralisiert, wobei das Verfahren zur Neutralisation nicht näher spezifiziert ist.

Gemäß der EP-A-0 205 674 werden saure Polymerisate bei Temperaturen im Bereich von 0 bis 100°C, vorzugsweise 5 bis 40°C hergestellt, deren pH durch nachträgliche Teilneutralisation des Hydrogels eingestellt wird. Die Neutralisation wird hierbei durch Zugabe des Gels in eine sehr stark verdünnte Natronlauge erzielt. Dieses Verfahren ist nachteilig, da bei der Trocknung durch die starke Verdünnung der Natronlauge große Wassermengen verdampft werden müssen.

In der EP-A-0 303 440 wird die Herstellung eines hydratisierten, vernetzten Gelpolymers beschrieben, bei dem die Säuregruppen enthaltenden Monomeren zu 10 bis 50 Mol-% neutralisiert sind, und welches durch Zugabe eines Neutralisationsmittels unter Nutzung der Scherkräfte der Rührblätter eines Reaktionsgefäßes mit einer Vielzahl von rotierenden Wellen auf den gewünschten Endneutralisationsgrad eingestellt wird. Nach diesem Verfahren wird zwar eine homogene Neutralisation erzielt, da ständig neue Oberfläche der Gelteilchen erzeugt wird, die Scherbelastung des Gels ist jedoch zu hoch und führt zum unerwünschten Anstieg der extrahierbaren Anteile.

Die EP-A-0 238 050 beansprucht ein Verfahren zur diskontinuierlichen Herstellung von vernetzten, feinteiligen, wasserabsorbierenden Polymerisaten, wobei die Polymerisation im Kneter durchgeführt wird und der Neutralisationsgrad der (Meth)acrylsäure zwischen 0 und 100 Mol-% liegt. Die Neutralisation des Polymerisationsansatzes zum gewünschten End-pH erfolgt ebenfalls in dem auch als Polymerisationsreaktor benutzen Kneter, und zwar entweder noch während der Polymerisation oder im Anschluß an die Polymerisationsreaktion. Auch hier wird zwar eine homogene Neutralisation erzielt, die aufgewendeten Scherkräfte sind jedoch zu hoch, so daß es zu einem unerwünschten Anstieg der Extrahierbaren kommt.

Nach der US-5 453 323 und der EP-A-0 530 438 werden ohne Neutralisation der Monomeren aus Acrylsäure zusammen mit wasserlöslichen Hydroxylgruppen enthaltenden Polymeren unter adiabatischen Bedingungen Polymerisatgele hergestellt, die anschließend in einem nicht weiter spezifizierten Fleischwolf zerkleinert werden. Zu diesem zerkleinerten Gel wird das Neutralisationsmittel gegeben und das Gemisch wird erneut gewolft. Anschließend erfolgt die Zugabe eines Nachvernetzungsmittels und eine erneute dreimalige Wolfung des Gels, um alle Reaktanden homogen in das Gel einzuarbeiten. Durch diese mehrmalige Wolfung des Gels findet eine unerwünschte Scherbelastung des Gels statt, die zu erhöhten Anteilen von Extrahierbaren führt.

Die EP-A-0 629 411 beschreibt die Polymerisation von Acrylsäure mit Vernetzern, wobei das erhaltene Gel anschließend mit einem Alkalisalz teilneutralisiert und durch Zugabe eines Vernetzungsmittels weiter vernetzt wird. Das Verfahren zur Neutralisation ist in der Schrift nicht näher spezifiziert, in einem Beispiel wird das Verkneten des Gels mit dem Neutralisationsmittel in einem Extruder erwähnt.

In der DE-A-195 29 348 ist zur Herstellung von superabsorbierenden Polymeren die adiabatische Polymerisation einer teilvorneutralisierten Monomerlösung beschrieben, wobei der Vorneutralisationsgrad der säuregruppentragenden Monomeren 5 bis 30 Mol-% beträgt. Die Neutralisation des sauren Gels erfolgt nach dessen Zerkleinerung in einfachen Mischaggregaten wie in einer rotierenden Trommel oder in einem Draismischer, wobei die wäßrige Lösung der Basen beispielsweise über Düsen oder Sprühlanzen eingetragen wird. Auf diese Weise wird zwar eine mechanische Schädigung des Polymergels vermieden, es kann aber keine homogene Neutralisation erzielt werden, da bei der Vermischung mit dem Neutralisationsmittel das Gel nicht aufgeschlossen wird. Die pH-Inhomogenitäten des Gels bewirken wiederum ein verschlechtertes Trocknungsverhalten, welches aus wirtschaftlichen Gründen unerwünscht ist.

Es bestand daher die Aufgabe, ein Verfahren zur Nachneutralisation von sauren Hydrogelen zu finden, bei dem eine homogene Neutralisation erzielt wird und bei dem gleichzeitig die Scherbelastung des Gels minimal ist, so daß kein unerwünschter Anstieg der extrahierbaren Anteile auftritt.

Demgemäß wurde ein Verfahren zur Herstellung wasserquellbarer hydrophiler Polymere durch Neutralisation des sauren Hydrogels mit einem Neutralisationsgrad von 0 - 40 Mol-% auf einen End-Neutralisationsgrad von 60 - 85 Mol-% durch Vermischen mit einem Neutralisationsmittel in einem Wolf mittels eines Systems aus Schnecke, rotierendem Messer, Stauzone und Lochscheibe gefunden, das dadurch gekennzeichnet ist, daß
- die Leistung des Wolfes von 1000 bis 6000 Wh/m³ beträgt
- das Hydrogel durch eine Zone mit einer Energiedissipationsdichte von 400 bis 800 W/l Mischvolumen geführt wird
- die mittlere Verweilzeit des Hydrogels im Wolf 5 bis 30 Sekunden beträgt
- die freie Lochfläche der Lochscheibe 20 bis 40 % beträgt.

Bevorzugt ist ein Verfahren zur Herstellung wasserquellbarer hydrophiler Polymere, gekennzeichnet durch
a) radikalische (Co)polymerisation von einem oder mehreren hydrophilen Monomeren oder Pfropf(co)polymerisation von einem oder mehreren hydrophilen Monomeren auf eine Pfropfgrundlage, wobei der durchschnittliche Neutralisationsgrad der säuregruppenhaltigen Monomeren 0 bis 40 Mol-% beträgt;
b) Grobzerkleinerung des sauren Hydrogels;
c) Neutralisation des sauren Hydrogels auf einen End-Neutralisationsgrad von 60 - 85 Mol-% durch Vermischen mit einem Neutralisationsmittel in einem Wolf mittels einem System aus Schnecke, rotierendem Messer, Stauzone und Lochscheibe, wobei
   - die Leistung des Wolfes 1000 bis 6000 Wh/m³ beträgt
   - das Hydrogel durch eine Zone mit einer Energiedissipationsdichte von 400 bis 800 W/l Mischvolumen geführt wird
   - die mittlere Verweilzeit des Hydrogels im Wolf 5 bis 30 Sekunden beträgt
   - die freie Lochfläche der Lochscheibe 20 bis 40 % beträgt;
d) Aufgabe der neutralisierten Hydrogelteilchen ohne weitere mechanische Scherbelastung auf einen Bandtrockner;
e) Trocknung der Hydrogelteilchen mit einem Bandtrockner; und
f) Mahlung und Siebung der getrockneten Hydrogelteilchen.

Das erfindungsgemäße Verfahren wird im folgenden erläutert.

Zur Herstellung der erfindungsgemäßen wasserquellbaren hydrophilen Polymeren geeignete hydrophile Monomere sind beispielsweise polymerisationsfähige Säuren, wie Acrylsäure, Methacrylsäure, Vinylsulfonsäure, Vinylphoshonsäure, Maleinsäure einschließlich deren Anhydrid, Fumarsäure, Itaconsäure, 2-Acrylamido-2-methylpropansulfonsäure, 2-Acrylamido-2-methylpropanphosphonsäure sowie deren Amide, Hydroxyalkylester und aminogruppen- oder ammoniumgruppenhaltige Ester und Amide. Des weiteren geeignet sind wasserlösliche N-Vinylamide oder auch Diallyldimethylammoniumchlorid.

Bevorzugte hydrophile Monomere sind Verbindungen der allgemeinen Formel (I) 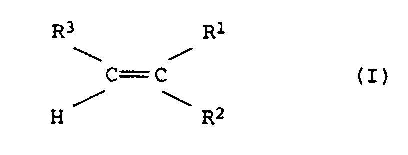 worin
- R¹: Wasserstoff, Methyl oder Ethyl,
- R²: eine Gruppe -COOR⁴, Hydroxysulfonyl, Phosphonyl, das mit (C₁-C₄)-Alkanol verestert sein kann, oder eine Gruppe der allgemeinen Formel (II) 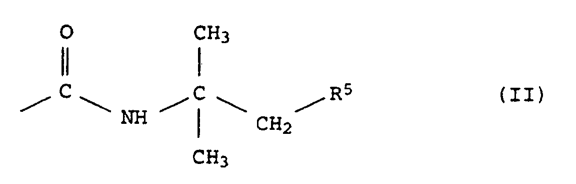
- R³: Wasserstoff, Methyl, Ethyl oder die Carboxylgruppe,
- R⁴: Wasserstoff, Amino-(C₁-C₄)-alkyl oder Hydroxy-(C₁-C₄)-alkyl und
- R⁵: Hydroxysulfonyl, Phosphonyl oder Carboxyl bedeuten.

Beispiele für (C₁-C₄)-Alkanole sind Methanol, Ethanol, n-Propanol oder n-Butanol.

Besonders bevorzugte hydrophile Monomere sind Acrylsäure und Methacrylsäure.

Sofern es sich bei den Monomeren um Säuren handelt, sind bis zu einem Anteil von 40 Gew.-% auch deren Alkalimetall- oder Ammoniumsalze als Comonomere geeignet.

Geeignete Pfropfgrundlagen können natürlichen oder synthetischen Ursprungs sein. Beispiele sind Stärke, Cellulose oder Cellulosederivate sowie andere Polysaccharide und Oligosaccharide, Polyvinylalkohol, Polyalkylenoxide, insbesondere Polyethylenoxide und Polypropylenoxide, sowie hydrophile Polyester. Geeignete Polyalkylenoxide haben beispielsweise die Formel (III) 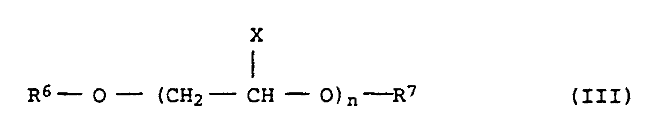 worin
- R⁶ und R⁷: unabhängig voneinander Wasserstoff, Alkyl, Alkenyl oder Aryl,
- X: Wasserstoff oder Methyl und
- n: eine ganze Zahl von 1 bis 10000 bedeuten.

Reste R⁶ und R⁷ sind beispielsweise lineares oder verzweigtes (C₁-C₁₀)-Alkyl sowie Methyl, Ethyl, Propyl, i-Propyl, n-Butyl, (C₂-C₆)-Alkenyl oder Aryl wie gegebenenfalls mit (C₁-C₄)-Alkyl substituiertes Phenyl.

R⁶ und R⁷ bedeuten bevorzugt Wasserstoff, (C₁-C₄)-Alkyl, (C₂-C₆)-Alkenyl oder Phenyl.

Die hydrophilen, hochquellfähigen Hydrogele sind bevorzugt vernetzt, d.h. sie enthalten Verbindungen mit mindestens zwei Doppelbindungen, die in das Polymernetzwerk einpolymerisiert sind.

Geeignete Vernetzer sind insbesondere Methylenbisacryl- bzw. - methacrylamid, Ester ungesättigter Mono- oder Polycarbonsäuren von Polyolen, wie Diacrylat oder Triacrylat, z.B. Butandiol- oder Ethylenglykoldiacrylat bzw. -methacrylat sowie Trimethylolpropantriacrylat, Allylverbindungen wie Allyl(meth)acrylat, Triallylcyanurat, Maleinsäurediallylester Polyallylester, Tetraallyloxyethan, Triallylamin, Tetraallylethylendiamin, Pentaerythritoltriallylester oder Allylester der Phosphorsäure sowie Vinylverbindungen wie Vinylacrylat, Divinyladipinat, Divinylbenzol und Vinylphosphonsäurederivate, wie sie beispielsweise in der EP-A-0 343 427 beschrieben sind.

Zur Auslösung der Polymerisation können energiereiche elektromagnetische Strahlen oder die üblichen chemischen Polymerisationsinitiätoren herangezogen werden, z.B. organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxiverbindungen wie Ammoniumpersulfat, Kaliumpersulfat oder Wasserstoffperoxid, gegebenenfalls in Kombination mit Reduktionsmitteln wie Natriumhydrogensulfit, und Eisen(II)-sulfat oder Redoxsystemen, welche als reduzierende Komponente eine aliphatische und aromatische Sulfinsäure, wie Benzolsulfinsäure und Toluolsulfinsäure oder Derivate dieser Säure enthalten, z.B. Mannichaddukte aus Sulfinsäure, Aldehyden und Aminoverbindungen.

Bevorzugt ist die Polymerisation in wäßriger Lösung nach der sogenannten Gel-Polymerisation unter Ausnutzung des Trommsdorff-Norrish-Effektes. Besonders bevorzugt ist, die Polymerisation in Ruhe ohne mechanische Durchmischung durchzuführen, so daß keine mechanischen Scherkräfte auf das sich bildende Hydrogel einwirken, die zu einer Erhöhung des Gehaltes an Extrahierbaren führen würden. Die Polymerisation kann hierbei sowohl diskontinuierlich, z.B. in einem zylindrischen Reaktor, oder kontinuierlich, z.B. durch Polymerisation auf einem Bandreaktor, durchgeführt werden.

Die Grobzerkleinerung der erhaltenen Hydrogele erfolgt mittels üblicher Reiß- und/oder Schneidwerkzeuge, z.B. durch die Wirkung einer Austragspumpe oder Austragsschnecke im Falle der Polymerisation in einem zylindrischen Reaktor oder durch eine Schneidwalze oder Schneidwalzenkombination im Falle der Bandpolymerisation.

Die sich anschließende Neutralisation des sauren Hydrogels erfolgt erfindungsgemäß, indem man das Hydrogel und das Neutralisationsmittel in einem Wolf mittels eines Systems aus Schnecke, rotierendem Messer, Stauzone und Lochscheibe mit einer Leistung von 1000 bis 6000 Wh/m³, bevorzugt von 2500 bis 5000 Wh/m³, aufgeschlossen und vermischt wird, wobei das Hydrogel durch eine Zone mit einer Energiedissipationsdichte von 400 bis 800 W/l Mischvolumen geführt wird. Das Verfahren arbeitet mit Verweilzeiten von 5 bis 30 Sekunden. Die Frequenz des rotierenden Messers beträgt 1 - 5 s⁻¹, bevorzugt 3 - 4 s⁻¹. Zur Reduzierung der Scherkräfte beim Mischvorgang im Stauraum vor der Lochscheibe des Apparates sind die Bohrungen der Lochscheibe konisch ausgeführt. Die freie Lochfläche der Lochscheibe beträgt 20 bis 40 %, vorzugsweise 25 bis 35 %, der Lochanfangdurchmesser 4 bis 16 mm, bevorzugt 8 bis 10 mm bei einer konischen Erweiterung unter einem Winkel von 8° bis 20°, vorzugsweise 10° bis 15°. Ein Wolf ähnelt apparativ einem Extruder übt aber geringere Scherkräfte aus.

Die beschriebene Konstruktion erlaubt die Kombination aus hoher Mischgüte und schonender mechanischer Beanspruchung des Gemisches aus Hydrogel und Neutralisationsmittel. Eine einstufige Behandlung erweist sich hierbei als absolut ausreichend für eine homogene Neutralisation, so daß ein mehrmaliges Wolfen des Gels vermieden wird, welches wiederum zu einem Anstieg der Scherbelastung des Gels führen würde, welcher unerwünscht ist.

Die Wahl des Neutralisationsmittels ist unkritisch, geeignete Neutralisationsmittel sind Alkalihydroxide, Ammoniak, aliphatische primäre und sekundäre Amine, Alkalicarbonate und Alkalihydrogencarbonate. Besonders bevorzugt sind Natriumhydroxid und Natriumcarbonat. Die Zugabe des Neutralisationsmittels kann sowohl in flüssiger Form, z.B. Natronlauge, in fester Form, z.B. Natriumcarbonat-Pulver oder gasförmig, z.B. Ammoniak, erfolgen.

Bei der erfindungsgemäßen Durchführung der Neutralisation ist es aufgrund der speziellen Konstruktion des Wolfes auch möglich, zusätzlich andere Reaktanden bzw. Stoffe mit dem zu neutralisieren den Polymergel zu vermischen. Durch dieses Verfahren wird ein mehrstufiges Wolfen vermieden, welches auf unerwünschte Weise zu einer mechanischen Scherbelastung des Gels führen würde.

So können dem Gel Reaktanden zugegeben werden, die mit freier Acrylsäure reagieren können, wie zum Beispiel Aminosäuren wie Cystein oder Lysin, Hydroxylamin und/oder seine Salze wie Hydrochlorid oder Sulfat, Hydrazin und/oder dessen Salze, Ozon oder schwefelhaltige Verbindungen mit reduzierender Wirkung, wie Alkalisulfite, -hydrogen-sulfite oder -disulfite, Natriumthiosulfat oder Mercaptoverbindungen.

Es können dem Gel auch Stoffe zugegeben werden, die mit den Carboxylgruppen des Hydrogels unter Vernetzung reagieren können. Beispiele für solche Stoffe sind mehrwertige Alkohole, mehrwertige Amine, Polyamidoamine und deren Umsetzungsprodukte mit Epichlorhydrin, Di- und Polyepoxide, Bis- und Polyaziridine, Bis- und Polyoxazoline, Di- und Polyisocyanate, Ethylencarbonat oder Oxazolidon.

Des weiteren ist es möglich, das Gel in dieser Stufe mit Feinanteilen superabsorbierender Polymere zu mischen, die beispielsweise bei der Herstellung von wasserquellbaren hydrophilen Hydrogelen bei der Mahlung und anschließenden Absiebung der getrockneten Hydrogele anfallen.

Zur Trocknung der Hydrogelteilchen sind verschiedene Verfahren bekannt. So kann die Trocknung beispielsweise nach dem Dünnfilm-Trockenverfahren, z.B. mit Hilfe eines Zweiachsen-Walzentrockners, nach dem Plattentrockenverfahren, gemäß dem die Hydrogel-polymerteilchen auf Platten in mehreren Schichten in einer Trockenkammer geladen werden, in der Heißluft zirkuliert, nach dem Drehtrommel-Verfahren mit Hilfe von Walzentrocknern oder nach dem Förderband-Verfahren, im folgenden auch als Bandtrocknung bezeichnet, erfolgen. Die Bandtrocknung, bei der mit Löcher versehene Horden eines Kreisförderers in einem Tunnel mit Trocknungsgut beladen und das Trocknungsgut während der Förderung durch Durchblasen von Heißluft durch die Hordenlöcher getrocknet wird, stellt das wirtschaftlichste Trocknungsverfahren für wasserquellbare hydrophile Hydrogele dar und ist daher bevorzugt. Die Trocknungsgeschwindigkeit des Trocknungsgutes wird bestimmt durch die Verdampfungsleistung, die angibt, wieviel kg Wasser aus dem zu trocknenden Produkt pro Quadratmeter Bandfläche pro Stunde verdampft werden. Diese Verdampfungsleistung sollte aus wirtschaftlichen Gründen möglichst hoch sein.

Die erfindungsgemäß neutralisierten Hydrogele, die bevorzugt mit zusätzlichen Reaktanden und/oder superabsorbierendem Feinkorn vermischt sind, weisen für die Bandtrocknung eine wirtschaftlich vorteilhafte Trocknungsgeschwindigkeit auf. Sie besitzen bei der Heißlufttrocknung bei 180°C und einer Luftgeschwindigkeit von 2 m/s eine Normverdampfungsleistung von mindestens 50 kg/m²h, bevorzugt von mindestens 70 kg/m²h, besonders bevorzugt von mindestens 80 kg/m²h.

In einem besonders bevorzugten Verfahren kann die Normverdampfungsleistung durch vorheriges Aufbringen eines Trennmittels auf die Hydrogelteilchen noch weiter gesteigert werden. Das Aufbringen der Trennmittel erfolgt hierbei ohne mechanische Belastung der Hydrogelpartikel durch Aufsprühen in dafür geeigneten Geräten, wie z.B. Drehrohr, Drais-Mischer, Pflugscharmischern wie z.B. Lödige-Mischer, Peterson-Kelly-Mischer, Kegel-Schnecken-Mischern etc.

Als Trennmittel geeignet sind nichtionische, anionische, kationische oder amphotere Tenside mit einem HLB-Wert größer gleich 3 (Definition des HLB-Wertes: siehe W.C. Griffin, J. Soc. Cosmetic Chem. 5 (1954) 249). Bevorzugt sind solche Tenside, die in Wasser löslich oder zumindest dispergierbar sind.

Geeignete nichtionische Tenside sind beispielsweise die Anlagerungsprodukte von Ethylenoxid, Propylenoxid oder Mischungen aus Ethylenoxid und Propylenoxid an Alkylphenole, aliphatische Alkohole, Carbonsäuren und Amine. Beispielsweise eignen sich mit Ethylenoxid und/oder Propylenoxid alkoxylierte C₈-C₁₂-Alkylphenole. Handelsübliche Produkte dieser Art sind beispielsweise Octylphenole bzw. Nonylphenole, die jeweils mit 4 bis 20 Mol Ethylenoxid pro Mol Phenol umgesetzt sind. Andere nichtionische Tenside sind ethoxylierte C₁₀-C₂₄-Fettalkohole oder ethoxylierte C₁₀-C₂₄-Fettsäuren sowie ethoxylierte C₁₀-C₂₄-Fettamine oder ethoxylierte C₁₀-C₂₄-Fettsäureamide. Außerdem eignen sich partiell mit C₁₀-C₂₄-Fettsäuren partiell veresterte mehrwertige C₃-C₆-Alkohole. Diese Ester können zusätzlich mit 2 bis 20 Mol Ethylenoxid umgesetzt sein. Als Fettalkohole, die zur Herstellung der Tenside alkoxyliert werden, eignen sich beispielsweise Palmitylalkohol, Stearylalkohol, Myristylalkohol, Laurylalkohol, Oxoalkohole sowie ungesättigte Alkohole, wie Oleylalkohol. Die Fettalkohole werden dabei zu einem solchen Grad ethoxyliert bzw. propoxyliert oder mit Ethylenoxid und Propylenoxid umgesetzt, daß die Reaktionsprodukte in Wasser löslich sind. Im allgemeinen setzt man 1 Mol der oben angegebenen Fettalkohole mit 2 bis 20 Mol Ethylenoxid und gegebenenfalls bis zu 5 Mol Propylenoxid so um, daß man Tenside erhält, die einen HLB-Wert von mehr als 8 haben.

C₃-C₆-Alkohole, die partiell verestert und gegebenenfalls ethoxyliert werden, sind beispielsweise Glycerin, Sorbit, Mannit und Pentaerythrit. Diese mehrwertigen Alkohole werden mit C₁₀-C₂₄-Fettsäuren, z.B. Ölsäure, Stearinsäure oder Palmitinsäure, partiell verestert. Die Veresterung mit den Fettsäuren erfolgt dabei höchstens bis zu einem solchen Grad, daß noch mindestens eine OH-Gruppe des mehrwertigen Alkohols unverestert bleibt. Geeignete Veresterungsprodukte sind beispielsweise Sorbitanmonooleat, Sorbitantristearat, Mannitmonooleat, Glycerinmonooleat und Glycerindioleat. Die genannten Fettsäureester mehrwertiger Alkohole, die noch mindestens eine freie OH-Gruppe enthalten, können zur Modifizierung noch mit Ethylenoxid, Propylenoxid oder Mischungen aus Ethylenoxid und Propylenoxid umgesetzt werden. Pro Mol Fettsäureester verwendet man vorzugsweise 2 bis 20 Mol der genannten Alkylenoxide. Der Ethoxylierungsgrad hat bekanntlich einen Einfluß auf den HLB-Wert der nichtionischen Tenside. Durch geeignete Wahl der Alkoxylierungsmittel und der Menge an Alkoxylierungsmittel kann man Tenside mit HLB-Werten in dem Bereich von 3 bis 20 in technisch einfacher Weise herstellen.

Eine weitere Gruppe geeigneter Substanzen sind Homopolymere des Ethylenoxids, Blockcopolymere von Ethylenoxid und Alkylenoxiden, vorzugsweise Propylenoxid sowie polyfunktionelle Blockcopolymere, die beispielsweise durch sequentielle Addition von Propylenoxid und Ethylenoxid an Diamine gebildet werden.

Des weiteren geeignet sind Alkylpolyglykoside, wie sie beispielsweise von der Fa. Henkel unter den Warenzeichen APG®, Glucopan® und Plantaren® vermarktet werden.

Die nichtionischen Tenside können entweder allein oder auch in Mischung miteinander verwendet werden.

Geeignete anionische Tenside sind C₈-C₂₄-Alkylsulfonate, die vorzugsweise in Form der Alkalisalze eingesetzt werden, C₈-C₂₄-Alkylsulfate, die vorzugsweise in Form der Alkali- oder Trialkanolammoniumsalze eingesetzt werden, wie z.B. Triethanolammoniumlaurylsulfat, Sulfobernsteinsäurediester, z.B. das Natriumsalz von Sulfobernsteinsäuredi-(2-ethylhexyl)-ester, Sulfobernsteinsäurehalbester, wie beispielsweise Natriumlaurylsulfosuccinat oder Dinatriumfettalkoholpolyglykolethersulfosuccinat, C₈-C₂₄-Alkylarylsulfonsäuren sowie die Schwefelsäurehalbester von Anlagerungsprodukten von Ethylenoxid an Alkylphenole oder Fettalkohole.

Beispiele für geeignete kationische Tenside sind die Salze von Fettaminen, z.B. Kokosfettammoniumacetat, quarternäre Fettsäureaminoester, z.B. Difettsäureisopropylesterdimethylammoniummethosulfat, quarternäre Fettsäureaminoamide, z.B. N-Undecylensäure-propylamido-N-trimethyl-ammoniummethosulfat, Anlagerungsprodukte von Alkylenoxiden an Fettamine bzw. Salze von Fettaminen, wie z.B. Pentaoxethylstearylammoniumacetat oder ethoxyliertes Methyloleinamin-Methosulfat sowie langkettige Alkylbenzyldimethylammoniumverbindungen, wie C₁₀-C₂₂-Alkylbenzyldimethylammoniumchlorid.

Beispiele für geeignete amphothere Tenside sind Verbindungen, die im gleichen Molekül mindestens ein quarternäres Ammoniumkation und mindestens ein Carboxylat- oder Sulfatanion tragen, wie beispielsweise Dimethylcarboxymethyl-Fettsäurealkylamidoammoniumbetaine oder 3-(3-Fettsäureamido-propyl)-dimethylammonium-2-hydroxypropan-sulfonate.

Die ionischen Tenside können allein oder auch in Mischung miteinander verwendet werden.

Die Tenside werden in Mengen von 0,001 bis 5, vorzugsweise 0,01 bis 2 Gew.-%, bezogen auf den Feststoffgehalt des zu trocknenden Polymerisatgels, angewendet. Bevorzugt ist hierbei der Einsatz von nichtionischen oder anionischen Tensiden, besonders bevorzugt der Einsatz von nichtionischen Tensiden, wie die partiell mit (C₁₀-C₂₄)-Fettsäuren veresterten mehrwertigen (C₃-C₆)-Alkohole, die mit 2 - 20 Mol Ethylenoxid umgesetzt sind oder die zuvor genannten Veresterungsprodukte, die nicht mit Ethylenoxid umgesetzt sind.

Des weiteren als Trennmittel geeignet sind Silicone wie z.B. Polysiloxane, in denen als organische Reste Methyl-, Ethyl, Propyl- oder Phenylgruppen, auch gleichzeitig mehrere dieser Reste, enthalten sind. Bevorzugt sind Polydimethylsiloxane und Polymethylphenylsiloxane, besonders bevorzugt Polydimethylsiloxane. Die Polysiloxane können Ketten- und ringförmige Polymere sein, bevorzugt sind solche mit linearem Aufbau, insbesondere Polydimethylsiloxane mit linearem Aufbau. Bevorzugt ist es weiterhin, die Silicone oder Polysiloxane in Form der handelsüblichen Produkte einzusetzen, die üblicherweise ein Substanzgemisch darstellen und auch modifizierte Silicone wie z.B. Aminosiloxane sein können. Als handelsübliche flüssige Silicone sind die allgemein als Siliconöle bezeichneten Produkte bevorzugt, insbesondere wiederum Siliconöle auf Basis Polydimethylsiloxan, speziell mit linearem Aufbau. Bevorzugt sind schließlich Siloxane mit einer Viskosität bei 25°C von 5 bis 20000 cSt, besonders bevorzugt solche mit 50 bis 350 cSt, ganz besonders bevorzugt solche mit 80 bis 120 cSt, speziell solche mit ca. 100 cSt.

Beispiele für andere, ebenfalls geeignete Trennmittel sind Hexadecanol, Octadecanol, Hexadecylacetat, Octadecylacetat, C₁₂-C₂₄-Fettsäuren und deren Salze wie z.B. Palimitinsäure und deren Salze oder Stearinsäure und deren Salze, Palmitinsäuremethylester, Butylstearat, Butyloleat, Hexylenglykol, Octamethylenglykol, Octadecan, Eicosan, handelsübliche Paraffinöle und Paraffine, die z.B. paraffinische, naphthenische und aromatische Kohlenwasserstoffe enthalten können, mit einem Schmelzpunkt von maximal 100°C und einem Dampfdruck von höchstens 0,1 mbar bei 20°C.

Eine weitere Gruppe von geeigneten Trennmitteln stellen Polyglykole und ihre Derivate dar, insbesondere Polyalkylenglykole und Polyalkylenglykolether, vor allem die Mono- und Dialkylether. Besonders bevorzugt sind Polyethylenglykole, Polypropylenglykole, Ethylenoxid/Propylenoxid-Mischpolymerisate, insbesondere Blockpolymerisate, Polyethylen-glykol- und Polypropylenglykol-mono- und - di(C₁-C₄)alkylether, insbesondere Methylether, aber auch Polyglykolether von höhermolekularen Fettalkoholen. Bevorzugt ist es wiederum, die Polyglykole und Polyglykolether in Form handelsüblicher Produkte einzusetzen, die üblicherweise ein Gemisch verschiedener Substanzen, insbesondere solcher mit verschiedenen Molekulargewichten, darstellen.

In einer bevorzugten Ausführung des Verfahrens wird als Trennmittel ein Neutralisationsmittel eingesetzt. Hier können alle Neutralisationsmittel eingesetzt werden, die auch für die Neutralisation des sauren Hydrogels im Wolf geeignet sind. Bevorzugt wird die Neutralisation im Wolf bis zu einem Neutralisationsgrad von mindestens 50 Gew.-%, bevorzugt von mindestens 55 Gew.-% und besonders bevorzugt von mindesten 60 Gew.-% eingestellt. Durch zusätzliche Behandlung des beispielsweise durch Aufsprühen des Neutralisationsmittels bzw. dessen wäßrige Lösung auf die Hydrogelpartikel, d.h. ohne mechanische Scherbelastung der Gelpartikel, wird der Neutralisationsgrad auf den gewünschten End-Neutralisationsgrad erhöht. Das Neutralisationsmittel im zweiten Schritt kann gleich oder verschieden dem Neutralisationsmittel im ersten Schritt sein. Bevorzugt wird für den zweiten Neutralisationsschritt wäßrige Natronlauge eingesetzt.

Die so erfindungsgemäß neutralisierten Hydrogele, die gegebenenfalls mit zusätzlichen Reaktanden und/oder superabsorbierendem Feinkorn vermischt sind, und anschließend in beschriebener Weise mit einem Trennmittel besprüht worden sind, weisen für die Bandtrocknung eine wirtschaftlich sehr vorteilhafte Trocknungsgeschwindigkeit auf. Sie besitzen bei der Heißlufttrocknung bei 180°C und einer Luftgeschwindigkeit von 2 m/s eine Normverdampfungsleistung von mindestens 90 kg/m²h, bevorzugt von mindestens 120 kg/m²h, und besonders bevorzugt von mindestens 140 kg/m²h.

Die Hydrogelteilchen werden anschließend getrocknet. Aus wirtschaftlicher Sicht ist hierbei die Bandtrocknung besonders bevorzugt. Neben zu optimierenden Faktoren wie Verteilung der Hydrogelteilchen auf dem Band, Schichthöhe der Hydrogelteilchen, Trocknungstemperatur bzw. -temperaturprofil, Luftfeuchte der Trocknerluft, Luftgeschwindigkeit, Luftverteilung, und Luftanströmrichtung besitzt die Struktur der Hydrogel-Partikel-Aufschichtung einen entscheidenden Einfluß auf die Trocknungsgeschwindigkeit. Die höchsten Trocknungsraten werden bei dem Vorliegen von lockeren, flockigen, separierten Gelteilchen erzielt, wie sie nach dem erfindungsgemäßen Verfahren erhalten werden.

Für die anschließende Mahlung der getrockneten Hydrogel-Teilchen ist es vorteilhaft, das Trockengut im letzten Abschnitt der Bandtrocknung auf Temperaturen < 70°C, bevorzugt < 60°C und besonders bevorzugt < 50°C abzukühlen. Die getrockneten, abgekühlten Hydrogelteilchen werden zunächst vorgebrochen, beispielsweise mit Hilfe eines Fingerbrechers (Vorzerkleinerer). Die so vorzerkleinerten Hydrogelteilchen werden dann gemahlen, wobei die Mahlung bevorzugt mit Hilfe eines oder mehreren hintereinandergeschalteten Walzenstühlen erfolgt, um den Anfall an Feinteilen möglichst klein zu halten. In einer besonders bevorzugten Ausführung erfolgt die Mahlung zweistufig, erst über einen Grobwalzenstuhl, dann über einen Feinwalzenstuhl, wobei letzterer wiederum ein- oder zweistufig sein kann. Bei der anschließenden Siebung wird die Korngrößenverteilung eingestellt, die in der Regel zwischen 100 und 1000 µm, bevorzugt zwischen 120 und 850 µm, liegt. Zu grobe Partikel können erneut der Mahlung unterworfen werden, zu feinteilige Partikel können dem Herstellungsprozeß zurückgeführt werden, beispielsweise durch Vermischung mit dem zu neutralisierenden Gel bei dem Nachneutralisationsschritt im Wolf, oder für getrennte Verwendungszwecke eingesetzt werden.

In einer bevorzugten Ausführung der Erfindung werden die Absorptionseigenschaften der so erhaltenen hydrophilen, hochquellfähigen Hydrogelen durch eine anschließende vorzugsweise kovalente Oberflächennachvernetzung noch weiter verbessert. Hierzu werden Verbindungen, die mit den Carboxylgruppen des Hydrogels unter Vernetzung reagieren können, vorzugsweise in Form einer wasserhaltigen Lösung auf die Oberfläche der Hydrogel-Partikel aufgebracht. Geeignete Nachvernetzungsmittel sind beispielsweise Di- oder Polyglycidylverbindungen wie Phosphonsäurediglycidylether oder Ethylenglykoldiglycidylether, Alkoxysilylverbindungen, Polyaziridine, Polyamine oder Polyamidoamine sowie deren Umsetzungsprodukte mit Epichlorhydrin, Polyole wie Ethylenglykol, 1,2-Propandiol, 1,4-Butandiol, Glycerin, Di- und Polyglycerin, Pentaerythrit, Sorbit, die Oxethylate dieser Polyole sowie deren Ester mit Carbonsäuren oder der Kohlensäure, Ethylencarbonat, Propylencarbonat, Oxazolidon, Bisoxazolin, Polyoxazoline, Di- und Polyisocyanate. Bei Bedarf können saure Katalysatoren wie beispielsweise p-Toluolsulfonsäure, Phosphorsäure, Borsäure oder Ammoniumdihydrogenphosphat zugesetzt werden.

Geeignete Mischaggregate zum Aufsprühen der Vernetzer-Lösung auf die Hydrogel-Partikel sind beispielsweise Patterson-Kelly-Mischer, DRAIS-Turbulenzmischer, Lödige-Mischer, Schneckenmischer, Tellermischer, Wirbelschichtmischer, Schugi-Mischer. Nach Aufsprühen der Vernetzer-Lösung kann ein Temperaturbehandlungsschritt folgen, bevorzugt in einem nachgeschalteten Trockner, bei einer Temperatur zwischen 80 und 230°C, bevorzugt 80 - 190°C, und besonders bevorzugt zwischen 100 und 160°C, über einen Zeitraum von 5 Minuten bis 6 Stunden, bevorzugt 10 Minuten bis 2 Stunden und besonders bevorzugt 10 Minuten bis 1 Stunde, wobei sowohl Spaltprodukte als auch Lösungsmittelanteile entfernt werden können.

In einer besonders bevorzugten Ausführung der Erfindung wird zusätzlich die Hydrophilie der Hydrogel-Partikeloberfläche durch Ausbildung von Metallkomplexen modifiziert. Die Bildung der Metallkomplexe auf der äußeren Schale der Hydrogel-Partikel erfolgt durch Aufsprühen von Lösungen zwei- oder mehrwertiger Metallsalz-Lösungen, wobei die Metall-Kationen mit den Carboxylgruppen des Hydrogels unter Ausbildung von Komplexen reagieren können. Beispiele für zwei- oder mehrwertige Metall-Kationen sind Mg²⁺, Ca²⁺, Al³⁺, Sc³⁺, Ti⁴⁺, Mn²⁺, Fe²⁺/Fe³⁺, Co²⁺, Ni²⁺, Cu⁺/Cu²⁺, Zn²⁺, Y³⁺, Zr⁴⁺, Ag⁺, La³⁺, Ce⁴⁺, Hf⁴⁺, und Au⁺/Au³⁺, bevorzugte Metall-Kationen sind Mg²⁺, Ca²⁺, Al³⁺, Ti⁴⁺, Zr⁴⁺ und La³⁺, und besonders bevorzugte Metall-Kationen sind Al³⁺, Ti⁴⁺ und Zr⁴⁺. Die Metall-Kationen können sowohl allein als auch im Gemisch untereinander eingesetzt werden. Von den genannten Metall-Kationen sind alle Metallsalze geeignet, die eine ausreichende Löslichkeit in dem zu verwendenden Lösungsmittel besitzen. Besonders geeignet sind Metallsalze mit schwach komplexierenden Anionen wie zum Beispiel Chlorid, Nitrat und Sulfat. Als Lösungsmittel für die Metallsalze können eingesetzt werden Wasser, Alkohole, DMF, DMSO sowie Mischungen dieser Komponenten. Besonders bevorzugt sind Wasser und Wasser/Alkohol-Mischungen wie zum Beispiel Wasser/Methanol oder Wasser/1,2-Propandiol.

Das Aufsprühen der Metallsalz-Lösung auf die Hydrogel-Partikel kann sowohl vor als auch nach der Oberflächennachvernetzung der Hydrogel-Partikel erfolgen. In einem besonders bevorzugten Verfahren erfolgt die Aufsprühung der Metallsalz-Lösung im gleichen Schritt mit dem Aufsprühen der Vernetzer-Lösung, wobei beide Lösungen getrennt nacheinander oder gleichzeitig über zwei Düsen aufgesprüht werden, oder Vernetzer- und Metallsalz-Lösung vereint über eine Düse aufgesprüht werden können.

Optional kann noch eine weitere Modifizierung der Hydrogel-Partikel durch Zumischung feinteiliger anorganischer Feststoffe, wie zum Beispiel Silica, Aluminiumoxid, Titandioxid und Eisen(II)-oxid erfolgen, wodurch die Effekte der Oberflächennachbehandlung noch weiter verstärkt werden. Besonders bevorzugt ist die Zumischung von hydrophilem Silica oder von Aluminiumoxid mit einer mittleren Größe der Primärteilchen von 4 bis 50 nm und einer spezifischen Oberfläche von 50 - 450 m²/g. Die Zumischung feinteiliger anorganischer Feststoffe erfolgt bevorzugt nach der Oberflächenmodifizierung durch Vernetzung /Komplexbildung, kann aber auch vor oder während diesen Oberflächenmodifizierungen durchgeführt werden.

Die erfindungsgemäßen Hydrogele zeichnen sich durch hervorragendes Absorptionsvermögen bei hoher Gelstärke und niedrigen Gehalten an Extrahierbaren aus und sind deshalb in hervorragender Weise als Absorptionsmittel für Wasser und wäßrige Flüssigkeiten, insbesondere von Körperflüssigkeiten, wie z.B. Urin oder Blut geeignet, beispielsweise in Hygieneartikeln wie z.B. Baby- und Erwachsenenwindeln, Damenbinden, Tampons und dergleichen. Sie können aber auch als Bodenverbesserungsmittel in Landwirtschaft und Gartenbau, als Feuchtigkeitsbindemittel bei der Kabelummantelung sowie zum Eindicken wäßriger Abfälle verwendet werden.

Beschreibung der in den Beispielen verwendeten Testmethoden:

### CRC (Centrifuge Retention Capacity):

Zur Bestimmung der CRC werden 0,2 g Hydrogel (Kornfraktion 106-850 µm) in einen 60 x 60 mm großen Teebeutel eingewogen, der anschließend verschweißt wird. Der Teebeutel wird dann in einen Überschuß von 0,9 gew.-%iger Kochsalz-Lösung gegeben (mindestens 1,25 1 Kochsalz-Lösung/1 g Hydrogel). Nach 20 Minuten Quellzeit wird der Teebeutel aus der Kochsalz-Lösung genommen und bei 250 g drei Minuten lang zentrifugiert. Durch Wägung des zentrifugierten Teebeutels wird die von dem Hydrogel festgehaltene Flüssigkeitsmenge ermittelt.

### Extrahierbare Anteile (16 h):

Zur Bestimmung der extrahierbaren Anteile werden 1 g Hydrogel (Kornfraktion 106-850 µm) in 200 ml 0,9 gew.-%ige Kochsalzlösung eingerührt. Das Becherglas wird abgedichtet und die Mischung 16 h lang gerührt. Danach wird durch einen 0,22 µm-Filter abfiltriert und der Gehalt an extrahierbaren Anteilen durch eine Säure-Base-Titration der Carboxylgruppen bestimmt (Titration mit 0,1 normaler NaOH bis pH 10, anschließend Titration mit 0,1 normaler HCl bis pH 2,7).

### AUL (Absorbency under Load):

Die Absorption unter Druck (AUL) wurde in bekannter Weise, wie beispielsweise in der EP-A-0 339 461 beschrieben bestimmt. Die AUL 70 bezieht sich hierbei auf die Messung der Absorption unter einer Druckbelastung von 70 g/cm², wobei die Flächenbelegung der Hydrogelpartikel (Kornfraktion 106 - 850 µm) in der Meßzelle 0,032 g/cm² beträgt.

### Gelsäulentest:

Die für den Gelsäulentest benötigte Apparatur besteht aus einer Glassäule mit einem Durchmesser von 2,6 cm und einer Mindestlänge von 40 cm, die am unteren Ende einen Siebboden (Fritte mit Porosität 0) und einen Auslaufhahn besitzt. Der Auslauf der Glassäule fließt in ein Becherglas, das auf einer Waage steht und dessen Gewicht kontinuierlich, zum Beispiel mit Hilfe eines Computers, registriert wird. Zur Durchführung des Gelsäulentests werden 1 g Hydrogel in 100 g 0,9 gew.-%iger NaCl-Lösung 5 Minuten lang gequollen. Das gequollene Gel wird in die Glassäule überführt. Zum Konditionieren des Gels werden 100 ml 0,9 gew.-%ige NaCl-Lösung zugegeben, der Auslaufhahn geöffnet und gewartet, bis die Flüssigkeit durch die gequollene Gelschicht ausläuft. Dann wird der Auslaufhahn geschlossen und erneut 100 g 0,9 gew.-%ige NaCl-Lösung zugegeben. Nach Öffnen des Auslaufhahnes wird die durchgeflossene Flüssigkeitsmenge in Abhängigkeit der Zeit registriert. Die nach 60 Sekunden durchgeflossene Flüssigkeitsmenge stellt den 60 s-Durchflußwert dar.

### Gelstärke:

Die Gelstärke wird mit einem Carri-Med-Stress-Rheometer mit einer Platte-Platte-Konfiguration gemessen. Zur Bestimmung der Gelstärke läßt man 1 g Hydrogel in 60 g 0,9 gew.-%iger Kochsalzlösung für 24 Stunden quellen und mißt anschließend an diesem gequollenen Gel den Speichermodul G' in Abhängigkeit von der Schubspannung bei einer Frequenz von 1 Hz. Der Plateauwert wird als Gelstärke angegeben.

### Bestimmung der Normverdampfungsleistung:

Die Bestimmung der Normverdampfungsleistung erfolgte mit Hilfe eines Konvektions-Bandtrockner-Simulators, wobei folgende Normbedingungen gewählt wurden:

| | |
|---|---|
| Normanfangsfeuchte des Hydrogels | 30 % |
| Normendfeuchte des Hydrogels | 5 % |
| Schütthöhe des Hydrogels | 40 mm |
| Anströmgeschwindigkeit | 2,0 m/s. |

### Beispiele:

### Polymerisation:

### Beispiel 1

In einem Ansatzkessel 1 wurde eine Mischung aus 367,7 kg entsalztem Wasser, 130,0 kg Acrylsäure, 1,0 kg Pentaerythritoltriallylether, 220 g 2,2'-Azobisamindinopropandihydrochlorid und 400 g Kaliumperoxodisulfat von Luftsauerstoff befreit und auf 4°C temperiert. In einem weiteren Ansatzkessel 2 wurde eine von Luftsauerstoff befreite Lösung von 40 g Ascorbinsäure in 20 kg Wasser hergestellt. Nach Fertigstellung der Lösungen wurde der Inhalt der beiden Ansatzkessel unter einem Druck von 1,5 bar im Stickstoffgegenstrom synchron in den Polymerisationsreaktor eingepreßt, wobei beide Lösungen mit Hilfe eines statischen Mischers vor Eintritt in den Reaktor gemischt wurden. Der Polymerisationsreaktor stellt ein 600 1-Rohr-Reaktor mit einem Durchmesser von 0,50 m dar, der sich am Ende konusartig verjüngt. Der Rohrreaktor wurde dann verschlossen und die Reaktionslösung wurde ohne Rühren stehengelassen, wobei durch einsetzende Polymerisation, in dessen Verlauf die Temperatur bis auf ca. 86°C ansteigt, ein festes Gel entsteht. Man ließ über Nacht auf Raumtemperatur abkühlen und preßte danach am Kopf des Reaktors einen Stickstoffdruck von 6 bar auf. Nach Öffnen des Absperrventils, welches sich am Ende des Konus des Reaktors befindet, konnte das Gel mit Hilfe einer Pumpe ausgetragen werden. Das Gel wurde hierbei durch die Wirkung der Pumpe zerkleinert und konnte in dieser Form direkt der weiteren Gelaufarbeitung unterzogen werden.

### Beispiel 2

Ein mit Aluminium beschichteter Tetrafluorethylen-Ethylen-Copolymerfilm wurde an der Oberfläche eines endlosen Bandes aus rostfreiem Stahl mit einer Breite von 450 mm und einer wirksamen Länge von 3000 mm so befestigt, daß die metallisierte Oberfläche mit der Bandoberfläche in Kontakt stand. Das endlose Band wurde in eine mit Stickstoff gefüllte Kammer, um die Sauerstoffkonzentration bei nicht mehr als 1 Vol.-% zu halten, eingeführt, während Sprüheinrichtungen so angeordnet sind, daß heißes oder kaltes Wasser auf die Rückseite des endlosen Bandes aufgesprüht werden konnte. Das endlose Band wurde mit einer Geschwindigkeit von 100 mm/min transportiert und Wasser von 15°C wurde nach oben auf das Band aufgesprüht.

In einem Ansatzkessel 1 wurden 5080 Gewichtsteile entsalztes Wasser vorgelegt, 669 Gewichtsteile Natriumbicarbonat darin suspendiert und langsam eine Mischung aus 2294 Gewichtsteilen Acrylsäure und 8 Gewichtsteilen Allylmethacrylat so zudosiert, daß ein Überschäumen der Reaktionslösung vermieden wurde, wobei sich diese auf eine Temperatur von ca. 3 - 5°C abkühlte. Bei einer Temperatur von 4°C wurden 2,2 Gewichtsteile 2,2'-Azobisamindinopropandihydrochlorid, gelöst in 20 Gewichtsteilen entsalztem Wasser, und 4 Gewichtsteile Kaliumperoxodisulfat, gelöst in 150 Gewichtsteilen entsalztem Wasser, nacheinander zugegeben und gut verrührt. In einem zweiten Ansatzkessel 2 wurde ein Lösung von 0,4 Gewichtsteilen Ascorbinsäure in 50 Gewichtsteilen entsalztem Wasser hergestellt.

Die Lösungen aus Ansatzkessel 1 und 2 wurden dann im Verhältnis 80:1 über einen statischen Mischer kontinuierlich mit einer Rate von 135 l/h auf ein Ende des sich bewegenden Bandes aufgebracht.

Unter den obengenannten Bedingungen betrug die Zeit, innerhalb der die Monomerlösung der Polymerisation auf dem sich bewegenden Band unterworfen wird, 30 Minuten und die Dicke der Monomerlösungsschicht auf dem Band betrug etwa 5 cm.

Am anderen Ende des endlosen Bandes wurde 30 Minuten nach Beginn der Zuführung der wäßrigen Monomerlösung ein Polymergel in Form eines Stranges mit einer Dicke von etwa 5 cm erhalten. Dieser Polymergelstrang wurde von der Bandoberfläche abgelöst und direkt in eine Schneideinrichtung vom Walzentyp eingeführt. Man erhielt so zerkleinerte Hydrogel-Teilchen, die direkt der weiteren Gelaufarbeitung unterworfen werden konnten.

### Gelaufarbeitung:

### Beispiel 3

Zur Neutralisation des in Beispiel 1 erhaltenen Hydrogels wurde ein Wolf gemäß Anspruch 1 verwendet, wobei die Leistung 4000 Wh/m³, die Frequenz des rotierenden Messer 3 s⁻¹, die Energiedissipationsdichte 600 W/l Mischvolumen, die Verweilzeit des Hydrogels im Wolf 20 s, die freie Lochfläche der Lochscheibe 32 % und der Lochanfangdurchmesser der Lochflächen 10 mm bei einer konischen Erweiterung unter einem Winkel von 12° betrug. Das Hydrogel wird zusammen mit einer 50 gew.-%igen Lösung an Natriumhydroxid dem oben beschriebenen Wolf zugeführt, wobei die Mengenanteile Hydrogel und Natronlauge so gewählt wurden, daß ein durchschnittlicher Neutralisationsgrad der Acrylsäureeinheiten des Hydrogels von 74 Mol-% erreicht wurde. Die pH-Homogenität des einmal gewolften Hydrogels wurde durch Aufsprühen von pH-Indikator-Lösung überprüft. Das gewolfte Hydrogel wurde dann unter Bestimmung der Normverdampfungsleistung Heißluft-getrocknet, wobei die Lufttemperatur 180°C betrug und die Luftgeschwindigkeit 2 m/s. Von dem getrockneten Hydrogel wurden die Anteile an Extrahierbaren bestimmt.

| pH-Homogenität des Gels | Normverdampfungsleistung | extrahierbare Anteile |
|---|---|---|
| homogen | 75 kg/m2h | 3,5 % |

### Vergleichsbeispiel 1

Es wurde wie in Beispiel 1 vorgegangen, die Wolfung findet jedoch in einem handelsüblichen Lebensmittelfleischwolf statt. Dieser Wolf war wie folgt charakterisiert:

| | |
|---|---|
| Leistung | 3500 Wh/m³ |
| Frequenz des rotierenden Messers | 4 s⁻¹ |
| Energiedissipationsdichte | 100 W/l Mischvolumen |
| Verweilzeit des Hydrogels im Wolf | 4 s |
| freie Lochfläche der Lochscheibe | 35 % |
| Lochanfangdurchmesser der Lochflächen | 10 mm (keine konische Erweiterung) |

| pH-Homogenität des Gels | Normverdampfungsleistung | extrahierbare Anteile |
|---|---|---|
| Inhomogen, ca. 30 % des Gels zu sauer (pH < 4,5) und ca. 30 % zu alkalisch (pH > 8,5) | 35 kg/m²h | 4,0 % |

Bei Benutzung eines nicht-erfindungsgemäßen Wolfes kann also durch einmaliges Wolfen keine homogene Neutralisation der Acrylsäure-Einheiten des Hydrogels erreicht werden. Das gewolfte Hydrogel wies eine deutlich niedrigere Normverdampfungsleistung auf.

### Vergleichsbeispiel 2

Es wurde wie in Vergleichsbeispiel 1 vorgegangen, es fand jedoch eine dreimalige Wolfung statt, um eine homogene Neutralisation zu erzielen.

| pH-Homogenität des Gels | Normverdampfungsleistung | extrahierbare Anteile |
|---|---|---|
| homogen | 40 kg/m²h | 10,5 % |

Durch mehrmaliges Wolfen konnte zwar eine homgene Neutralisation erzielt werden, es findet aber ein deutlicher Anstieg der Extrahierbaren statt. Das gewolfte Hydrogel wies eine deutlich niedrigere Normverdampfungsleistung auf als in Beispiel 3.

### Beispiel 4

Das in Beispiel 2 erhaltene Hydrogel wurde zusammen mit 0,7 Gew.-% (bezogen auf Acrylsäure) Festsubstanz eines handelsüblichen kationischen Polyamidoaminharzes (KYMENE 557H® der Hercules Corp., USA), 20 Gew.-% (bezogen auf Acrylsäure) eines superabsorbierenden Feinkorns, wobei 90 % der Partikel kleiner als 120 µm war, sowie mit einer 50 gew.-%igen Lösung an Natriumhydroxid einem Wolf zugeführt, der wie folgt charakterisiert ist:

| | |
|---|---|
| Leistung | 5000 Wh/m³ |
| Frequenz des rotierenden Messers | 3 s⁻¹ |
| Energiedissipationsdichte | 750 W/l Mischvolumen |
| Verweilzeit des Hydrogels im Wolf | 25 s |
| freie Lochfläche der Lochscheibe | 30 % |
| Lochanfangdurchmesser der Lochflächen | 8 mm (mit konischer Erweiterung unter einem Winkel von 15°), |

wobei die Mengenanteile Hydrogel und Natronlauge so gewählt wurden, daß ein durchschnittlicher Neutralisationsgrad der Acrylsäureeinheiten des Hydrogels von 70 Mol-% erreicht wurde. Die pH-Homogenität des einmal gewolften Hydrogels wurde durch Aufsprühen von pH-Indikator-Lösung überprüft. Das gewolfte Hydrogel wurde dann unter Bestimmung der Normverdampfungsleistung Heißluft-getrocknet, wobei die Lufttemperatur 180°C betrug und die Luftgeschwindigkeit 2 m/s. Von dem getrockneten Hydrogel wurden die Anteile an Extrahierbaren bestimmt.

| pH-Homogenität des Gels | Normverdampfungsleistung | extrahierbare Anteile |
|---|---|---|
| homogen | 79 kg/m²h | 1,5 % |

Auch bei Zusatz von superabsorbierendem Feinkorn und einem zusätzlichen Reaktanden konnte bereits bei einmaliger Wolfung eine homogene Neutralisation erzielt werden.

### Beispiel 5

Das in Beispiel 1 erhaltene Hydrogel wurde zusammen mit einer 50 gew.-%igen Lösung an Natriumhydroxid dem in Beispiel 3 beschriebenen Wolf zugeführt, wobei die Mengenanteile Hydrogel und Natronlauge so gewählt wurden, daß ein durchschnittlicher Neutralisationsgrad der Acrylsäureeinheiten des Hydrogels von 74 Mol-% erreicht wird. Anschließend wurden die Hydrogelpartikel in einem kontinuierlichen Drais-Mischer mit verschiedenen Trennmitteln gemäß nachfolgender Tabelle besprüht. Man erhielt sehr lockere, flockige Gele mit separierten Gelteilchen. Die Hydrogele wurden dann unter Bestimmung der Normverdampfungsleistung heißluftgetrocknet, wobei die Lufttemperatur 180°C betrug und die Luftgeschwindigkeit 2 m/s.

| Trennmittel | Gew.-% Trennmittel bezogen auf Acrylsäure | Normverdampfungsleistung |
|---|---|---|
| Sorbitanmonococoat | 0,2 % | 110 kg/m²h |
| Hostapur® SAS 30 | 0,3 % | 125 kg/m²h |
| Trennmittel | Gew.-% Trennmittel bezogen auf Acrylsäure | Normverdampfungsleistung |
| C₁₂-C₁₄-Alkylbenzyl-di-methylammoniumchlorid | 0,25 % | 95 kg/m²h |
| Ampholyt® JB 130/K | 0,20 % | 105 kg/m²h |
| PEG 300 | 0,35 % | 115 kg/m²h |
| MPG 350 | 0,35 % | 130 kg/m²h |
| PIONIER® 2024 | 0,1 % | 120 kg/m²h |
| BAYSILONE® M 100 | 0,1 % | 150 kg/m²h |
| GENAPOL® PF 40 | 0,15 % | 115 kg/m²h |
| Palmitinsäure, Natriumsalz | 0,2 % | 100 kg/m²h |
| Butyloleat | 0,05 % | 140 kg/m²h |
| Hostapur® SAS 30 (Handelsprodukt der Clariant GmbH) ist ein durch Sulfoxidation von n-Paraffinen hergestellte Mischung von n-Alkansulfonaten | | |
| Ampholyt® JB 130/K (Handelsprodukt der Hüls AG) ist ein Coco-amidopropyl-Betain | | |
| PEG 300 ist Polyethylenglykol mit einem durchschnittlichen Molekulargewicht von 300 g/mol | | |
| MPG 350 ist Methylpolyethylenglykol mit einem durchschnittlichen Molekulargewicht von 350 g/mol | | |
| PIONIER® 2024 (Handelsprodukt der Fa. Hansen & Rosenthal) ist ein technisches Paraffinöl | | |
| BAYSILONE® M 100 (Handelsprodukt der Bayer AG) ist ein Polydimethylsiloxan | | |
| GENAPOL® PF 40 (Handelsprodukt der Clariant GmbH) ist ein Ethylenoxid-Propylenoxid-Blockcopolymer. | | |

Der Vergleich mit Beispiel 3 zeigte, daß durch die der Wolfung anschließende Nachbehandlung der Gelpartikel eine Steigerung der Normverdampfungsleistung erreicht wurde.

### Beispiel 6

Das in Beispiel 2 erhaltene Hydrogel wurde zusammen mit pulverförmigem Natriumcarbonat dem in Beispiel 4 beschriebenen Wolf zugeführt, wobei die Mengenanteile Hydrogel und Natriumcarbonat so gewählt wurden, daß ein durchschnittlicher Neutralisationsgrad der Acrylsäureeinheiten des Hydrogels von 60 Mol-% erreicht wird. Anschließend wurden die Hydrogelpartikel in einem kontinuierlichen Drehrohr-Mischer mit verschiedenen Neutralisationsmitteln gemäß Tabelle besprüht, so daß sich ein End-Neutralisationsgrad der Acrylsäureeinheiten des Hydrogels von 70 Mol-% ergab. Man erhielt sehr lockere, flockige Gele mit separierten Gelteilchen. Die Hydrogele wurden dann unter Bestimmung der Normverdampfungsleistung Heißluft-getrocknet, wobei die Lufttemperatur 180°C betrug und die Luftgeschwindigkeit 2 m/s.

| Neutralisationsmittel | Normverdampfungsleistung |
|---|---|
| 50 gew.-%ige Natronlauge | 125 kg/m²h |
| 50 gew.-%ige Kalilauge | 140 kg/m²h |
| Natriumcarbonat (15 gew.-%ige wäßrige Lösung) | 115 kg/m²h |
| Kaliumcarbonat (40 gew.-%ige wäßrige Lösung) | 130 kg/m²h |
| Ammoniak (25 gew.-%ige wäßrige Lösung) | 95 kg/m²h |

Der Vergleich mit Beispiel 4 zeigte, daß durch die der Wolfung anschließende Nachbehandlung der Gelpartikel eine Steigerung der Normverdampfungsleistung erreicht wurde.

### Oberflächennachvernetzung:

### Beispiel 7

In einem Lödige-Pflugscharmischer von 100 1 Inhalt werden 35 kg Hydrogel-Pulver, hergestellt gemäß Beispiel 3, vorgelegt. Im Verlauf von 5 bis 10 Minuten wurde eine Lösung von 28 g Ethylenglykoldiglycidylether, 1170 g Wasser und 580 g 1,2-Propandiol aufgedüst. Es wurde auf 120°C Produkttemperatur angeheizt und 60 Minuten bei dieser Temperatur gehalten, um das Lösungsmittel wieder abzudestillieren. Anschließend wird abgekühlt, das Produkt ausgetragen und die Kornfraktion 120 - 850 µm abgesiebt. Man erhielt ein Produkt, das gekennzeichnet war durch folgende physikalischen Daten, alle gemessen in 0,9 gew.-%iger Kochsalzlösung:

| | |
|---|---|
| CRC = | 33 g/g |
| AUL 70 = | 25 g/g |
| Gelstärke = | 2500 Pa |
| Extrahierbare Anteile 16 h = | 1,6 % |
| Gelsäule, 60 s-Durchflußwert = | 90 g |

### Beispiel 8

In einem Petterson & Kelly-Mischer von 10 1 Inhalt wurden 6 kg Hydrogelpulver, hergestellt gemäß Beispiel 4, vorgelegt. Unter Mischen wurde im Verlauf von 5 Minuten eine Lösung aus 12 g Bisoxazolin, 9 g Aluminiumsulfat, 225 g i-Propanol und 225 g Wasser aufgedüst und 1 Minute nachgemischt. Das Produkt wurde anschließend im Trockenschrank 30 Minuten bei 185°C nachgetempert. Es war gekennzeichnet durch folgende physikalischen Daten, alle gemessen in 0,9 gew.-%iger Kochsalzlösung:

| | |
|---|---|
| CRC = | 27 g/g |
| AUL 70 = | 26 g/g |
| Gelstärke = | 4000 Pa |
| Extrahierbare Anteile 16 h = | 0,4 % |
| Gelsäule, 60 s-Durchflußwert = | 98 g |

### Beispiel 9

In einem Lödige-Pflugscharmischer von 100 1 Inhalt wurden 35 kg Hydrogelpulver, hergestellt gemäß Beispiel 5, unter Verwendung von Hostapur® SAS 30 als Trennmittel, vorgelegt. Im Verlauf von 5 bis 10 Minuten wurde eine Lösung von 105 g KYMENE 557H®, 1400 g Wasser und 1400 g Methanol aufgedüst. Es wurde auf 150°C Produkttemperatur angeheizt und 45 Minuten bei dieser Temperatur gehalten, um das Lösungsmittel wieder abzudestillieren. Anschließend wird abgekühlt, das Produkt ausgetragen, mit 0,2 Gew.-% hydrophilem Silica (Aerosil 200) abgemischt und die Kornfraktion 120 - 850 µm abgesiebt. Man erhielt ein Produkt, das gekennzeichnet ist durch folgende physikalischen Daten, alle gemessen in 0,9 gew.-%iger Kochsalzlösung:

| | |
|---|---|
| CRC = | 31 g/g |
| AUL 70 = | 26 g/g |
| Gelstärke = | 3000 Pa |
| Extrahierbare Anteile 16 h = | 1,2 % |
| Gelsäule, 60 s-Durchflußwert = | 96 g |

### Beispiel 10

In einem Petterson & Kelly-Mischer von 10 1 Inhalt wurden 6 kg Hydrogel-Pulver, hergestellt gemäß Beispiel 6, unter Verwendung von Natronlauge als zweites Neutralisationsmittel, vorgelegt. Unter Mischen wurde im Verlauf von 5 Minuten eine Lösung aus 12 g Oxazolidon, 180 g Ethanol und 270 g Wasser aufgedüst und 2 Minuten nachgemischt. Das Produkt wurde anschließend im Trockenschrank 30 Minuten bei 195°C nachgetempert. Es war gekennzeichnet durch folgende physikalischen Daten, alle gemessen in 0,9 gew.-%iger Kochsalzlösung:

| | |
|---|---|
| CRC = | 28 g/g |
| AUL 70 = | 26 g/g |
| Gelstärke = | 4200 Pa |
| Extrahierbare Anteile 16 h = | 0,5 % |
| Gelsäule, 60 s-Durchflußwert = | 99 g |

Die nach Beispiel 7 bis 10 erhaltenen oberflächennachvernetzten Hydrogele zeichnen sich durch hervorragendes Absorptionsvermögen bei hoher Gelstärke, hoher Gelpermeabilität und niedrigen Gehalten an Extrahierbaren aus und sind deshalb in hervorragender Weise als Absorptionsmittel für Wasser und wäßrige Flüssigkeiten, insbesondere von Körperflüssigkeiten, wie z.B. Urin oder Blut geeignet, beispielsweise in Hygieneartikeln wie z.B. Baby- und Erwachsenenwindeln, Damenbinden, Tampons und dergleichen.

## Patentansprüche

1. Verfahren zur Herstellung wasserquellbarer hydrophiler Polymere durch Neutralisation des sauren Hydrogels mit einem Neutralisationsgrad von 0 - 40 Mol-% auf einen End-Neutralisationsgrad von 60 - 85 Mol-% durch Vermischen mit einem Neutralisationsmittel in einem Wolf mittels eines Systems aus Schnecke, rotierendem Messer, Stauzone und Lochscheibe, **dadurch gekennzeichnet, daß**
- die Leistung des Wolfes von 1000 bis 6000 Wh/m³ beträgt
- das Hydrogel durch eine Zone mit einer Energiedissipationsdichte von 400 bis 800 W/l Mischvolumen geführt wird
- die mittlere Verweilzeit des Hydrogels im Wolf 5 bis 30 Sekunden beträgt
- die freie Lochfläche der Lochscheibe 20 bis 40 % beträgt.

2. Verfahren zur Herstellung wasserquellbarer hydrophiler Polymere, **gekennzeichnet durch**
a) radikalische (Co)polymerisation von einem oder mehreren hydrophilen Monomeren oder Pfropf(co)polymerisation von einem oder mehreren hydrophilen Monomeren auf eine Pfropfgrundlage, wobei der durchschnittliche Neutralisationsgrad der säuregruppenhaltigen Monomeren 0 bis 40 Mol-% beträgt
b) Grobzerkleinerung des sauren Hydrogels
c) Neutralisation des sauren Hydrogels auf einen End-Neutralisationsgrad von 60 - 85 Mol-% **durch** Vermischen mit einem Neutralisationsmittel in einem Wolf mittels eines Systems aus Schnecke, rotierendem Messer, Stauzone und Lochscheibe, wobei
- die Leistung des Wolfes 1000 bis 6000 Wh/m³ beträgt
- das Hydrogel **durch** eine Zone mit einer Energiedissipationsdichte von 400 bis 800 W/l Mischvolumen geführt wird
- die mittlere Verweilzeit des Hydrogels im Wolf 5 bis 30 Sekunden und
- die freie Lochfläche der Lochscheibe 20 bis 40 % beträgt
d) Aufgabe der neutralisierten Hydrogelteilchen ohne weitere mechanische Scherbelastung auf einen Bandtrockner
e) Trocknung der Hydrogelteilchen mit einem Bandtrockner und
f) Mahlung und Siebung der getrockneten Hydrogelteilchen.

3. Verfahren nach Anspruch 1 oder 2, **dadurch gekennzeichnet, daß** die Polymerisation ohne Durchmischung erfolgt.

4. Verfahren nach den Ansprüchen 1 bis 3, **dadurch gekennzeichnet, daß** hydrophile Monomere Verbindungen der allgemeinen Formel (I) 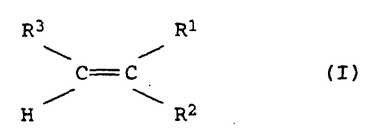 worin
R¹ Wasserstoff, Methyl oder Ethyl,
R² eine Gruppe -COOR⁴, Hydroxysulfonyl, Phosphonyl, die mit (C₁-C₄)-Alkanol veresterte Phosphonylgruppe oder eine Gruppe der Formel (II) 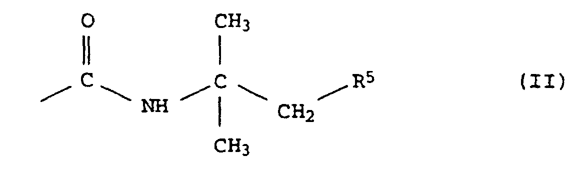
R³ Wasserstoff, Methyl, Ethyl oder die Carboxyl,
R⁴ Wasserstoff, Amino-(C₁-C₄)-alkyl oder Hydroxy-(C₁-C₄)-alkyl und
R⁵ Hydroxysulfonyl, Phosphonyl oder Carboxyl bedeuten.

5. Verfahren nach einem oder mehreren der Ansprüche 1 bis 4, **dadurch gekennzeichnet, daß** geeignete Pfropfgrundlagen Stärke, Cellulose, Cellulosederivate, Polyvinylalkohol, Polyalkylenoxid, Polyethylenoxid, Polypropylenoxid und hydrophile Polyester sind.

6. Verfahren nach einem oder mehreren der Ansprüche 1 bis 5, **dadurch gekennzeichnet, daß** die (Co)polymerisation der hydrophilen Monomeren in Anwesenheit von Vernetzern durchgeführt wird.

7. Verfahren nach einem oder mehreren der Ansprüche 1 bis 6, **gekennzeichnet dadurch, daß** die neutralisierten Gelteilchen nach der Wolfung bei der Heißlufttrocknung bei 180°C und einer Luftgeschwindigkeit von 2 m/s eine Normverdampfungsleistung von mindestens 50 kg/m²h aufweisen.

8. Verfahren nach einem oder mehreren der Ansprüche 1 bis 7, **gekennzeichnet dadurch, daß** bei der Neutralisation des Gels bei der Wolfung zusammen mit dem Neutralisationsmittel noch ein oder mehrere anderer Reaktionsstoffe, die mit freier Acrylsäure und/oder mit den Carboxylgruppen des Hydrogels reagieren können, und/oder Feinkorn von wasserquellbaren hydrophilen Polymeren zugesetzt wird.

9. Verfahren nach einem oder mehreren der Ansprüche 1 bis 8, **gekennzeichnet dadurch, daß** vor Auflage der Hydrogelteilchen auf den Bandtrockner diese ohne mechanische Scherbelastung mit einem Trennmittel besprüht werden.

10. Verfahren nach einem oder mehreren der Ansprüche 1 bis 9, **dadurch gekennzeichnet, daß** die Neutralisation in zwei Schritten durchgeführt wird, wobei der erste Neutralisationsschritt im Wolf stattfindet und der zweite Neutralisationsschritt durch Aufbringen des Neutralisationsmittels ohne mechanische Scherbelastung auf die Hydrogelpartikel erfolgt.

11. Verfahren nach einem oder mehreren der Ansprüche 1 bis 10, **dadurch gekennzeichnet, daß** die Mahlung der getrockneten Hydrogelteilchen in einem oder mehreren hintereinandergeschalteten Walzenstühlen erfolgt.

12. Verfahren nach einem oder mehreren der Ansprüche 1 bis 11, **dadurch gekennzeichnet, daß** die getrockneten und gemahlenen Hydrogelteilchen kovalent oberflächennachvernetzt werden.

13. Verfahren nach einem oder mehreren der Ansprüche 1 bis 12, **dadurch gekennzeichnet, daß** die Oberfläche der getrockneten und gemahlenen Hydrogelpartikel durch die Bildung von Metallkomplexen modifiziert wird.

14. Wasserquellbare hydrophile Polymere erhältlich gemäß dem Verfahren der Ansprüche 1 bis 13.

15. Verwendung der Polymere gemäß Anspruch 14 als Absorptionsmittel für Wasser und wäßrige Flüssigkeiten.

16. Verwendung gemäß Anspruch 15 zur Absorption von Körperflüssigkeiten, insbesondere in Hygieneartikeln, wie z. B. Windeln, Tampons oder Damenbinden.

## Claims

1. The process for preparing water-swellable hydrophilic polymers by neutralization of the acidic hydrogel having a degree of neutralization of 0 - 40 mol% to an ultimate degree of neutralization of 60 - 85 mol% by mixing with a neutralizing agent in a mincer comprising a system of screw, rotating blade, restricted flow zone and breaker plate, wherein
- the mincer has a power output of from 1000 to 6000 Wh/m³
- the hydrogel passes through a zone having an energy dissipation density of from 400 to 800 W/l of mixing volume
- the average residence time of the hydrogel in the mincer is from 5 to 30 seconds
- the breaker plate has an open area of from 20 to 40%.

2. A process for preparing water-swellable hydrophilic polymers, which comprises
a) free-radically (co)polymerizing one or more hydrophilic monomers or graft (co)polymerizing one or more hydrophilic monomers onto a grafting base, the average degree of neutralization of the acid-functional monomers being from 0 to 40 mol%,
b) coarsely comminuting the acidic hydrogel,
c) neutralization of the acidic hydrogel to an ultimate degree of neutralization of 60 - 85 mol% by mixing with a neutralizing agent in a mincer comprising a system of screw, rotating blade, restricted flow zone and breaker plate, wherein
- the mincer has a power output of from 1000 to 6000 Wh/m³
- the hydrogel passes through a zone having an energy dissipation density of from 400 to 800 W/l of mixing volume
- the average residence time of the hydrogel in the mincer is from 5 to 30 seconds and
- the breaker plate has an open area of from 20 to 40%,
d) placing the neutralized hydrogel particles without further mechanical shearing stress onto a belt dryer
e) drying the hydrogel particles using a belt dryer and
f) grinding and sieving the dried hydrogel particles.

3. The process of claim 1 or 2, wherein the polymerization is effected without mixing.

4. The process of any of claims 1 to 3, wherein hydrophilic monomers are compounds of the general formula (I) 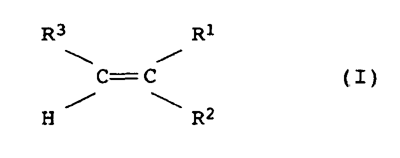 where
R¹ is hydrogen, methyl or ethyl,
R² is -COOR⁴, hydroxysulfonyl, phosphonyl, a (C₁-C₄)-alkanol-esterified phosphonyl group or a group of the formula (II) 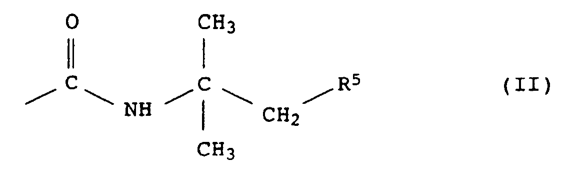
where
R³ is hydrogen, methyl, ethyl or carboxyl,
R⁴ is hydrogen, amino-(C₁-C₄)-alkyl or hydroxy-(C₁-C₄)-alkyl, and
R⁵ is hydroxysulfonyl, phosphonyl or carboxyl.

5. The process of one or more of claims 1 to 4, wherein suitable grafting bases are selected from the group consisting of starch, cellulose, cellulose derivatives, polyvinyl alcohol, polyalkylene oxide, polyethylene oxide, polypropylene oxide and hydrophilic polyesters.

6. The process of one or more of claims 1 to 5, wherein the (co)polymerizing of the hydrophilic monomers is effected in the presence of crosslinkers.

7. The process of one or more of claims 1 to 6, wherein the neutralized and minced gel particles have a standardized evaporation rate of at least 50 kg/m²h on hot air drying at 180°C and an air velocity of 2 m/s.

8. The process of one or more of claims 1 to 7, wherein, as well as the neutralizing agent, one or more other reactive materials capable of reacting with free acrylic acid and/or with the carboxyl groups of the hydrogel and/or water-swellable hydrophilic polymer fines are added during the neutralization of the gel in the course of mincing.

9. The process of one or more of claims 1 to 8, wherein the hydrogel particles are sprayed with a release agent without mechanical shearing stress before being placed on the belt dryer.

10. The process of one or more of claims 1 to 9, wherein the neutralization is carried out in two steps, the first neutralization step taking place in the mincer and the second neutralization step being effected by applying the neutralizing agent to the hydrogel particles without mechanical shearing stress.

11. The process of one or more of claims 1 to 10, wherein the grinding of the dried hydrogel particles is effected in one or more successive roll mills.

12. The process of one or more of claims 1 to 11, wherein the dried and ground hydrogel particles are covalently surface postcrosslinked.

13. The process of one or more of claims 1 to 12, wherein the surface of the dried and ground hydrogel particles is modified by the formation of metal complexes.

14. Water-swellable hydrophilic polymer obtainable by the process of any of claims 1 to 13.

15. The method of using polymer as claimed in claim 14 as an absorbent for water and aqueous fluids.

16. The method of claim 15 for absorbing body fluids, especially in hygiene articles such as diapers, tampons or sanitary napkins.

## Revendications

1. Procédé de préparation de polymères hydrophiles gonflables dans l'eau par neutralisation de l'hydrogel acide présentant un degré de neutralisation de 0-40 moles % à un degré de neutralisation final de 60-85 moles %, par mélange avec un agent de neutralisation dans un hachoir au moyen d'un système constitué d'une vis, d'un couteau rotatif, d'une zone de retenue et d'un disque perforé, **caractérisé en ce que**
- la puissance du hachoir est de 1000 à 6000 Wh/m³,
- l'hydrogel est guidé à travers une zone avec une densité de dissipation d'énergie de 400 à 800 W/l de volume de mélange,
- la durée de séjour moyenne de l'hydrogel dans le hachoir est de 5 à 30 secondes,
- la surface perforée libre du disque perforé est de 20 à 40%.

2. Procédé de préparation de polymères hydrophiles gonflables dans l'eau, **caractérisé par**
a) une (co)polymérisation radicalaire d'un ou de plusieurs monomères hydrophiles ou une (co)polymérisation de greffage d'un ou de plusieurs monomères hydrophiles sur une base de greffage, le degré de neutralisation moyen des monomères contenant des groupes acides étant de 0 à 40 moles %,
b) une fragmentation grossière de l'hydrogel acide,
c) une neutralisation de l'hydrogel acide à un degré de neutralisation final de 60-85 moles % par mélange d'un agent de neutralisation dans un hachoir au moyen d'un système constitué d'une vis, d'un couteau rotatif, d'une zone de retenue et d'un disque perforé,
- la puissance du hachoir étant de 1000 à 6000 Wh/m³,
- l'hydrogel étant guidé au travers d'une zone avec une densité de dissipation d'énergie de 400 à 800 W/l de volume de mélange,
- la durée de séjour moyenne de l'hydrogel dans le hachoir étant de 5 à 30 secondes, et
- la surface perforée libre du disque perforé étant de 20 à 40%,
d) une alimentation des particules d'hydrogel neutralisées à un séchoir à bande, sans autre sollicitation mécanique par cisaillement,
e) un séchage des particules d'hydrogel à l'aide d'un séchoir à bande, et
f) un broyage et un tamisage des particules d'hydrogel séchées.

3. Procédé suivant l'une des revendications 1 et 2, **caractérisé en ce que** la polymérisation a lieu sans mélange intime.

4. Procédé suivant l'une des revendications 1 à 3, **caractérisé en ce que** des monomères hydrophiles sont des composés de la formule générale (I) : 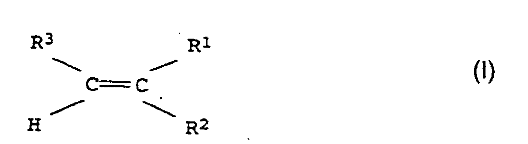 dans laquelle
R¹ représente de l'hydrogène, du méthyle ou de l'éthyle,
R² représente un groupe -COOR⁴, hydroxysulfonyle, phosphonyle, le groupe phosphonyle estérifié par un alcanoi en C₁-C₄ ou un groupe de la formule (II) : 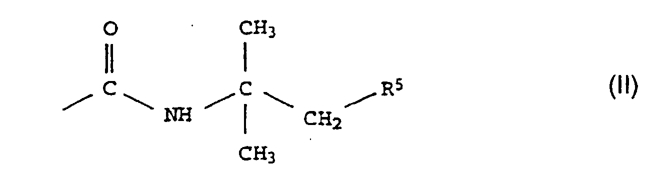
R³ représente de l'hydrogène, du méthyle, de l'éthyle ou le radical carboxyle,
R⁴ représente de l'hydrogène ou un radical aminoalkyle en C₁-C₄ ou hydroxyalkyle en C₁-C₄, et
R⁵ représente de l'hydroxysulfonyle, du phosphonyle ou du carboxyle.

5. Procédé suivant une ou plusieurs des revendications 1 à 4, **caractérisé en ce que** des bases de greffage appropriées sont de l'amidon, de la cellulose, des dérivés de cellulose, de l'alcool polyvinylique, de l'oxyde de polyalkylène, de l'oxyde de polyéthylène, de l'oxyde de polypropylène et des polyesters hydrophiles.

6. Procédé suivant une ou plusieurs des revendications 1 à 5, **caractérisé en ce que** la (co)polymérisation des monomères hydrophiles est effectuée en présence d'agents de réticulation.

7. Procédé suivant une ou plusieurs des revendications 1 à 6, **caractérisé en ce que** les particules de gel neutralisées après le hachage présentent, lors du séchage par de l'air chaud à 180°C et à une vitesse d'air de 2 m/s, un puissance d'évaporation standard d'au moins 50 kg/m²h.

8. Procédé suivant une ou plusieurs des revendications 1 à 7, **caractérisé en ce que**, lors de la neutralisation du gel au cours du hachage conjointement à l'agent de neutralisation, on ajoute encore une ou plusieurs autres substances réactionnelles, qui peuvent réagir avec de l'acide acrylique libre et/ou les groupes carboxyle de l'hydrogel, et/ou des grains fins de polymères hydrophiles gonflables dans l'eau.

9. Procédé suivant une ou plusieurs des revendications 1 à 8, **caractérisé en ce que**, avant l'alimentation des particules d'hydrogel au séchoir à bande, celles-ci sont aspergées par un agent séparateur, sans sollicitation mécanique par cisaillement.

10. Procédé suivant une ou plusieurs de revendications 1 à 9, **caractérisé en ce que** la neutralisation est effectuée en deux étapes, la première étape de neutralisation ayant lieu dans le hachoir et la deuxième étape de neutralisation étant effectuée par application de l'agent de neutralisation sur les particules d'hydrogel, sans sollicitation mécanique par cisaillement.

11. Procédé suivant une ou plusieurs des revendications 1 à 10, **caractérisé en ce que** le broyage des particules d'hydrogel séchées a lieu dans un ou plusieurs moulins à cylindres montés successivement.

12. Procédé suivant une ou plusieurs des revendications 1 à 11, **caractérisé en ce que** les particules d'hydrogel séchées et broyées sont ensuite réticulées en surface de manière covalente.

13. Procédé suivant un ou plusieurs des revendications 1 à 12, **caractérisé en ce que** la surface des particules d'hydrogel séchées et broyées est modifiée par la formation de complexes métalliques.

14. Polymères hydrophiles gonflables dans l'eau, que l'on peut obtenir selon le procédé des revendications 1 à 13.

15. Utilisation des polymères suivant la revendication 14, comme agents d'absorption de l'eau et des liquides aqueux.

16. Utilisation suivant la revendication 15, pour l'absorption de liquides corporels, en particulier dans des articles d'hygiène, comme par exemple des langes, des tampons ou des bandes hygiéniques.
